# EUROPEAN PATENT APPLICATION

(11) **EP 4 538 271 A1**
(43) Date of publication of application: **16.04.2025**
(21) Application number: 23819044.1
(22) Date of filing: 05.06.2023
(51) Int. Cl.: C07D 413/12, A61K 31/353, A61K 31/553, A61P 11/06, A61P 11/08, A61P 19/02, A61P 3/10, A61P 37/00, A61P 13/12

(54) **POLYMORPH OF BENZO[C]CHROMAN COMPOUND, PREPARATION METHOD THEREFOR AND USE THEREOF**

(30) Priority: 07.06.2022 CN 202210639488
(71) Applicant: Reistone Biopharma Company Limited, Shanghai 201210 (CN)
(72) Inventor: SUN, Longwei, Shanghai 201210 (CN); HOU, Yanting, Shanghai 201210 (CN); ZHU, Jiajun, Shanghai 201210 (CN); YANG, Guang, Shanghai 201210 (CN); HAO, Xin, Shanghai 201210 (CN); LUO, Zhiyang, Shanghai 201210 (CN)
(74) Representative: Dragotti & Associati S.R.L.
(86) International application number: PCT/CN2023/098209
(87) International publication number: WO 2023/236879

(57) **Abstract**

Disclosed in the present invention are a polymorph of a benzo[c]chroman compound, a preparation method therefor and use thereof. The present invention particularly provides a polymorph of a compound represented by formula I, a preparation method therefor and use thereof. The compound represented by formula I is used as a cathepsin C inhibitor, and a crystalline form of the compound and a pharmaceutical composition comprising the crystalline form can be used for treating asthma, obstructive pulmonary diseases, bronchiectasis, ANCA-related vasculitis, psoriasis, α1-antitrypsin deficiency, lupus nephritis, diabetes, inflammatory bowel diseases, rheumatic arthritis, rhinosinusitis, hidradenitis suppurativa or cancers.

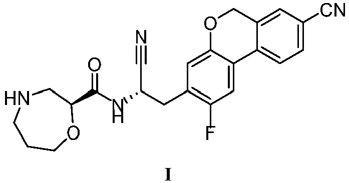

## Description

### FIELD OF THE INVENTION

The present disclosure belongs to the field of pharmaceutical technology, and relates to polymorphs of a benzo[c]chromane compound and preparation methods and uses thereof.

### BACKGROUND OF THE INVENTION

Cathepsins are a class of proteolytic enzymes widely present in lysosomes of various tissue cells. According to their structures and catalytic types, cathepsins are divided into three classes: serine proteases (cathepsins A and G), aspartic proteases (cathepsins D and E), and cysteine proteases. Among them, cysteine proteases are the largest family of cathepsins and include 11 proteases: cathepsins B, C, F, H, K, L, O, S, W, V, and Z.

Cathepsin C is also known as dipeptidyl peptidase I or "DPP1". DPP1 is constitutively expressed in many tissues with the highest levels in the lung, kidney, liver, and spleen. Several recently published studies have described the role played by cathepsin C in certain inflammatory processes. For example, Adkison et al., J Clin Invest. 2002 Feb; 109(3):363-71; Tinh et al., Archives of Biochemistry and Biophysics. 2002 403:160-170, it can be seen from these studies that cathepsin C is co-expressed in granules with certain serine proteases, and functions to process the precursor forms of these proteases into active forms, which are then released from inflammatory cell granules recruited to sites of inflammation. Once activated, these proteases have numerous functions, including the degradation of various extracellular matrix components, which together can propagate tissue damage and chronic inflammation.

WO 2004/110988 relates to certain nitrile derivatives and the use thereof as DPP1 inhibitors.

WO 2009/074829 relates to peptidyl nitriles and the use thereof as DPP1 inhibitors.

WO 2010/128324 relates to α-aminoamide nitriles and the use thereof as DPP1 inhibitors.

WO 2012/119941 relates to peptidyl nitrile compounds and the use thereof as DPP1 inhibitors.

WO 2013/041497 relates to N-[1-cyano-2-(phenyl)ethyl]-2-azabicyclo[2.2.1]heptane-3-carboxamide and the use thereof as a DPP1 inhibitor.

WO 2001/096285 and WO 2003/048123 relate to β-aminoamide nitriles having inhibitory activity against cysteine proteases.

WO 2015/110826 relates to α-aminoamide nitriles and the use thereof as DPP1 inhibitors.

WO 2022/117059 provides a cathepsin C inhibitor, chemically named (*S*)-*N*-((*S*)-1-cyano-2-(8-cyano-2-fluoro-6*H*-benzo[*c*]chromen-3-yl)ethyl)-1,4-oxazepane-2-carboxamide, with the structure shown in formula I.

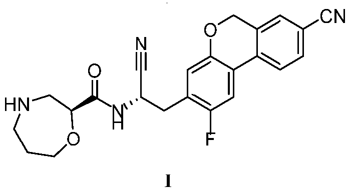

The crystal form of a pharmaceutically active ingredient often affects the chemical stability of a drug, and different crystallization and storage conditions may lead to changes in the crystal structure of a compound, sometimes accompanied by production of other crystal forms. In general, an amorphous drug product does not have a regular crystal structure, and often has other defects such as poor product stability, fine precipitation, difficult filtration, easy agglomeration, poor flowability and the like. The polymorphism of a drug has different requirements on product storage, production, and scaleup. Therefore, it is necessary to conduct in-depth research on crystal forms of the aforementioned compound so as to improve various properties of the aforementioned compound.

### SUMMARY OF THE INVENTION

The present disclosure provides crystal form A of a compound of formula I, wherein the X-ray powder diffraction pattern thereof represented by diffraction angle 2θ angle has characteristic peaks at 8.910, 10.273, 15.650, 18.617, 17.869 and 19.526, wherein the error range of the 2θ angle is ±0.20.

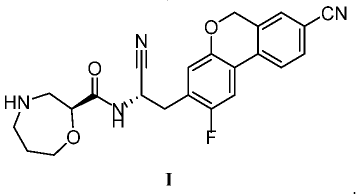

In some embodiments, provided is the crystal form A of the compound of formula I, wherein the X-ray powder diffraction pattern thereof represented by diffraction angle 2θ angle has characteristic peaks at 8.910, 10.273, 13.730, 15.650, 17.869, 18.617, 19.526 and 23.452, wherein the error range of the 2θ angle is ±0.20.

In some embodiments, provided is the crystal form A of the compound of formula I, wherein the X-ray powder diffraction pattern thereof represented by diffraction angle 2θ angle has characteristic peaks at 8.910, 10.273, 13.730, 15.650, 17.869, 18.617, 19.526, 20.785, 22.193, 23.452, 25.049 and 26.590, wherein the error range of the 2θ angle is ±0.20.

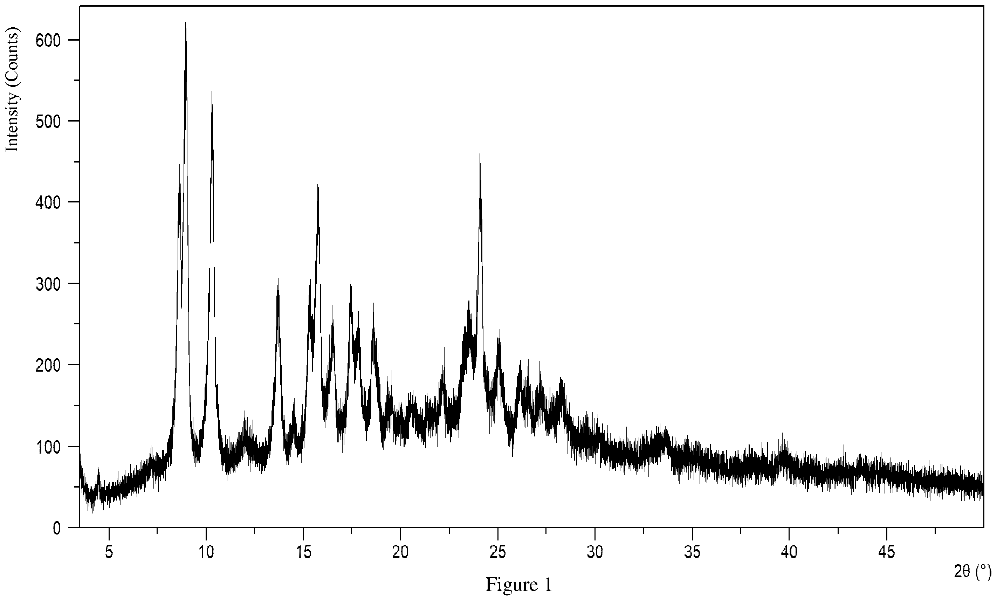

In some embodiments, provided is the crystal form A of the compound of formula I, wherein the X-ray powder diffraction pattern thereof represented by diffraction angle 2θ angle is as shown in Figure 1.

In some embodiments, provided is the crystal form A of the compound of formula I, wherein the differential scanning calorimetry (DSC) spectrum thereof has endothermic peaks at 116°C and 208°C, with the error range of ±2°C.

The present disclosure also provides a method for preparing crystal form A of the compound of formula I, comprising the steps of:
a) mixing the compound of formula I with Sovent A;
b) performing stirring;
wherein the Solvent A is selected from the group consisting of water, C₁₋₄ alcohol solvent, and mixed solvent of water and C₁₋₄ alcohol solvent, and the C₁₋₄ alcohol solvent preferably is methanol and ethanol.

In some embodiments, in the method for preparing crystal form A of the compound of formula I, each milligram of the compound of formula I is mixed withfrom 0.01 to 0.05 ml of the Solvent A, more preferably each milligram of the compound of formula I is mixed withfrom 0.01 to 0.03 ml of the Solvent A.

In some embodiments, in the method for preparing crystal form A, the temperature in the stirring is room temperature or 50°C.

In some embodiments, in the method for preparing crystal form A, the mixed solvent of water and alcohol solvent is a mixed solvent of water and methanol, wherein the molar ratio of water to methanol is from 0.10 to 0.95, preferably 0.14, 0.26, 0.37, 0.47, 0.57, 0.66, 0.74, 0.82 or 0.90.

The present disclosure also provides crystal form B of the compound of formula I above, wherein the X-ray powder diffraction pattern thereof represented by diffraction angle 2θ angle has characteristic peaks at 6.770, 11.201, 12.989, 14.931 and 20.817, wherein the error range of the 2θ angle is ±0.20.

In some embodiments, provided is the crystal form B of the compound of formula I, wherein the X-ray powder diffraction pattern thereof represented by diffraction angle 2θ angle has characteristic peaks at 6.770, 11.201, 12.989, 14.931, 20.817 and 25.710, wherein the error range of the 2θ angle is ±0.20.

In some embodiments, provided is the crystal form B of the compound of formula I, wherein the X-ray powder diffraction pattern thereof represented by diffraction angle 2θ angle has characteristic peaks at 6.770, 11.201, 12.989, 14.931, 20.817, 25.710, 31.066, 32.046 and 32.913, wherein the error range of the 2θ angle is ±0.20.

In some embodiments, provided is the crystal form B of the compound of formula I, wherein the X-ray powder diffraction pattern thereof represented by diffraction angle 2θ angle has characteristic peaks at 6.770, 11.201, 12.989, 14.931, 15.851, 17.228, 20.817, 22.313, 22.773, 25.710, 28.192, 31.066, 32.046 and 32.913, wherein the error range of the 2θ angle is ±0.20.

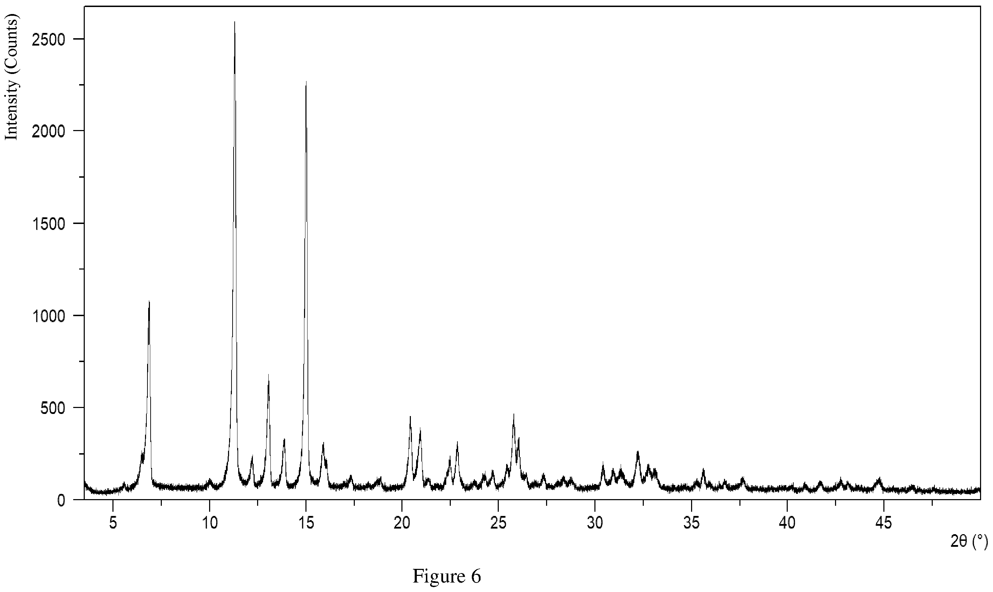

In some embodiments, provided is the crystal form B of the compound of formula I, wherein the X-ray powder diffraction pattern thereof represented by diffraction angle 2θ angle is as shown in Figure 6.

In some embodiments, provided is the crystal form B of the compound of formula I, wherein the DSC spectrum thereof has an endothermic peak at 210°C, with the error range of ±2°C.

The present disclosure also provides a method for preparing crystal form B of the compound of formula I, comprising the steps of:
a) mixing the compound of formula I above with Sovent B;
b) performing stirring;
wherein the Solvent B is selected from the group consisting of nitromethane and acetonitrile.

In some embodiments, in the method for preparing crystal form B of the compound of formula I, each 100 mg of the compound of formula I is mixed with from 0.5 to 5 ml of the Solvent B, more preferably each 100 mg of the compound of formula I is mixed withfrom 0.5 to 2 ml of the Solvent B.

In some embodiments, in the method for preparing crystal form B of the compound of formula I, each 100 mg of the compound of formula I is mixed with 1 ml of the Solvent B.

In some embodiments, in the method for preparing crystal form B, the temperature in the stirring is room temperature or 50°C.

The present disclosure also provides crystal form C of the compound of formula I above, wherein the X-ray powder diffraction pattern thereof represented by diffraction angle 2θ angle has characteristic peaks at 7.385, 10.171, 12.687, 15.902 and 19.645, wherein the error range of the 2θ angle is ±0.20.

In some embodiments, provided is the crystal form C of the compound of formula I, wherein the X-ray powder diffraction pattern thereof represented by diffraction angle 2θ angle has characteristic peaks at 6.012, 7.385, 8.275, 10.171, 12.687, 15.120, 15.902 and 19.645, wherein the error range of the 2θ angle is ±0.20.

In some embodiments, provided is the crystal form C of the compound of formula I, wherein the X-ray powder diffraction pattern thereof represented by diffraction angle 2θ angle has characteristic peaks at 6.012, 7.385, 8.275, 10.171, 12.687, 15.120, 15.902, 19.645, 25.539 and 26.382, wherein the error range of the 2θ angle is ±0.20.

In some embodiments, provided is the crystal form C of the compound of formula I, wherein the X-ray powder diffraction pattern thereof represented by diffraction angle 2θ angle has characteristic peaks at 6.012, 7.385, 8.275, 10.171, 12.687, 13.720, 15.120, 15.902, 16.842, 19.645, 20.739, 25.539, 26.382, 27.272 and 30.887, wherein the error range of the 2θ angle is ±0.20.

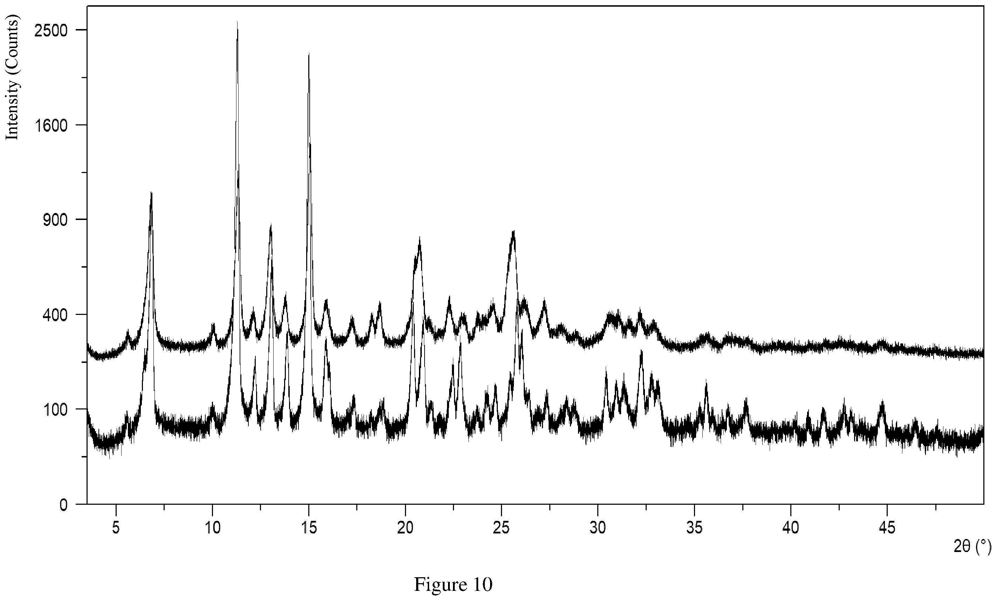

In some embodiments, provided is the crystal form C of the compound of formula I, wherein the X-ray powder diffraction pattern thereof represented by diffraction angle 2θ angle is as shown in Figure 10.

In some embodiments, provided is the crystal form C of the compound of formula I, wherein the DSC spectrum thereof has an endothermic peak at 209°C, with the error range of ±2°C.

The present disclosure also provides a method for preparing crystal form C of the compound of formula I, comprising the steps of:
a) mixing the compound of formula I with Sovent C;
b) performing stirring;
wherein the Solvent C is selected from the group consisting of N,N-dimethylformamide, acetone, butanone, tetrahydrofuran and 1,2-dimethoxyethane.

In some embodiments, in the method for preparing crystal form C of the compound of formula I, each 100 mg of the compound of formula I is mixed withfrom 0.5 to 5 ml of the Solvent C, more preferably each 100 mg of the compound of formula I is mixed with from 0.5 to 2 ml of the Solvent C, most preferably each 100 mg of the compound of formula I is mixed with 1 ml of the Solvent C.

In some embodiments, in the method for preparing crystal form C, the temperature in the stirring is room temperature or 50°C.

The present disclosure also provides crystal form D of the compound of formula I above, wherein the X-ray powder diffraction pattern thereof represented by diffraction angle 2θ angle has characteristic peaks at 8.477, 10.451, 13.569, 15.146, 16.335 and 17.275, wherein the error range of the 2θ angle is ±0.20.

In some embodiments, provided is the crystal form D of the compound of formula I, wherein the X-ray powder diffraction pattern thereof represented by diffraction angle 2θ angle has characteristic peaks at 8.477, 10.451, 13.569, 15.146, 16.335, 17.275 and 18.775, wherein the error range of the 2θ angle is ±0.20.

In some embodiments, provided is the crystal form D of the compound of formula I, wherein the X-ray powder diffraction pattern thereof represented by diffraction angle 2θ angle has characteristic peaks at 8.477, 10.451, 13.569, 15.146, 16.335, 17.275, 18.775, 20.298, 23.990, 26.006 and 28.135, wherein the error range of the 2θ angle is ±0.20.

In some embodiments, provided is the crystal form D of the compound of formula I, wherein the X-ray powder diffraction pattern thereof represented by diffraction angle 2θ angle has characteristic peaks at 8.477, 10.451, 13.569, 15.146, 16.335, 17.275, 18.775, 20.298, 23.093, 23.990, 24.921, 26.006, 27.047, 28.135 and 33.461, wherein the error range of the 2θ angle is ±0.20.

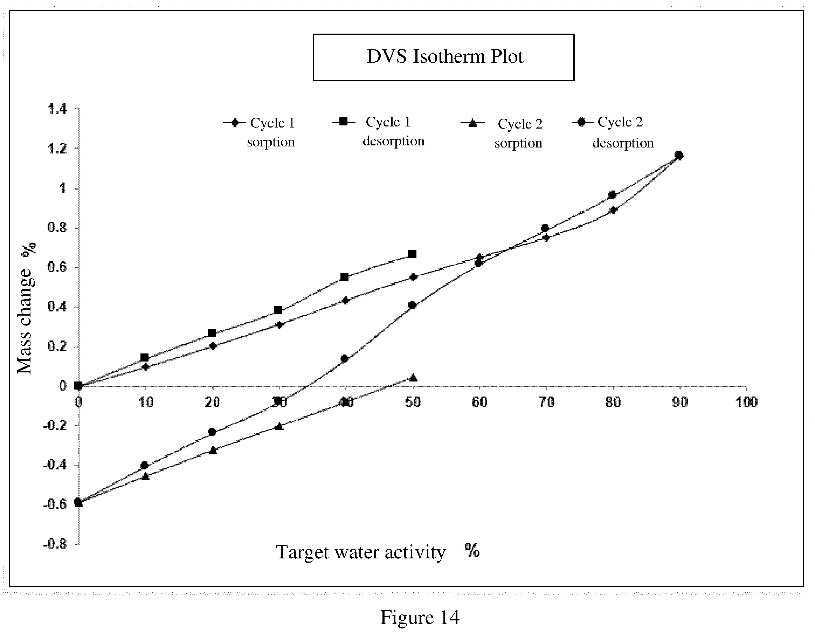

In some embodiments, provided is the crystal form D of the compound of formula I, wherein the X-ray powder diffraction pattern thereof represented by diffraction angle 2θ angle is as shown in Figure 14.

In some embodiments, provided is the crystal form D of the compound of formula I, wherein the DSC spectrum thereof has an endothermic peak at 211°C, with the error range of ±2°C.

The present disclosure also provides a method for preparing crystal form D of the compound of formula I, comprising the steps of:
a) mixing the compound of formula I with Sovent D;
b) performing stirring;
wherein the Solvent D is selected from the group consisting of ethyl acetate, 1,2-xylene, toluene, 1,4-dioxane and hexane.

In some embodiments, in the method for preparing crystal form D of the compound of formula I, each 100 mg of the compound of formula I is mixed withfrom 0.5 to 5 ml of the Solvent D, more preferably each 100 mg of the compound of formula I is mixed withfrom 0.5 to 2 ml of the Solvent D, preferably each 100 mg of the compound of formula I is mixed with 1 ml of the Solvent D.

In some embodiments, in the method for preparing crystal form D, the temperature in the stirring is room temperature or 50°C.

In some embodiments, the method of the present disclosure further comprises the step(s) of performing filtering, washing and/or drying.

The present disclosure also provides crystal form E of the compound of formula I above, wherein the X-ray powder diffraction pattern thereof represented by diffraction angle 2θ angle has characteristic peaks at 8.685, 15.433, 16.654, 17.526 and 18.779, wherein the error range of the 2θ angle is ±0.20.

In some embodiments, provided is the crystal form E of the compound of formula I, wherein the X-ray powder diffraction pattern thereof represented by diffraction angle 2θ angle has characteristic peaks at 8.685, 10.681, 13.773, 14.600, 15.433, 16.654, 17.526 and 18.779, wherein the error range of the 2θ angle is ±0.20.

In some embodiments, provided is the crystal form E of the compound of formula I, wherein the X-ray powder diffraction pattern thereof represented by diffraction angle 2θ angle has characteristic peaks at 8.685, 10.681, 13.773, 14.600, 15.433, 16.654, 17.526, 18.779, 19.393, 20.610, 21.653, 23.319 and 24.151, wherein the error range of the 2θ angle is ±0.20.

In some embodiments, provided is the crystal form E of the compound of formula I, wherein the X-ray powder diffraction pattern thereof represented by diffraction angle 2θ angle has characteristic peaks at 7.233, 8.685, 10.681, 13.773, 14.600, 15.433, 16.654, 17.526, 18.779, 20.610, 21.653, 23.319, 24.151, 25.111 and 26.192, wherein the error range of the 2θ angle is ±0.20.

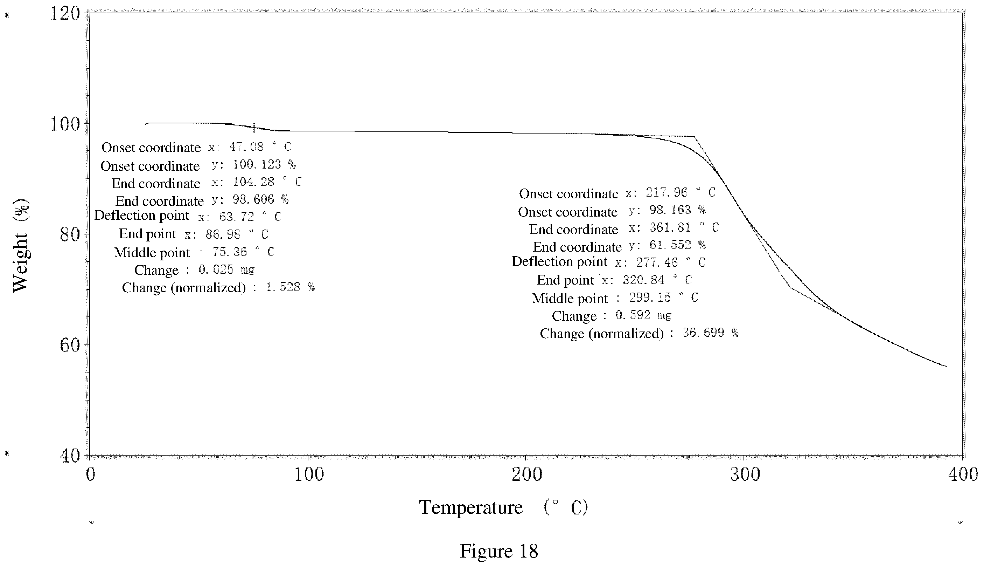

In some embodiments, provided is the crystal form E of the compound of formula I, wherein the X-ray powder diffraction pattern thereof represented by diffraction angle 2θ angle is as shown in Figure 18.

The present disclosure also provides a method for preparing crystal form E of the compound of formula I, comprising a step of drying or heating the compound of formula I, wherein the temperature for drying or heating is preferably from 100 to 200°C.

In some embodiments, the method for preparing crystal form E of the compound of formula I comprises a step of heating the compound of formula I to 100°C or higher, preferably heating the compound of formula I to 120°C or higher, and more preferably heating the compound of formula I to 150°C or higher.

The present disclosure also provides a pharmaceutical composition comprising the above crystal form A, crystal form B, crystal form C, crystal form D or crystal form E, and a pharmaceutically acceptable excipient.

The present disclosure also provides a method for preparing the pharmaceutical composition described above, comprising a step of mixing the above crystal form A, crystal form B, crystal form C, crystal form D or crystal form E with a pharmaceutically acceptable excipient.

The present disclosure also relates to a use of the above crystal form A, crystal form B, crystal form C, crystal form D or crystal form E or the above composition, in the preparation of a medicament for preventing and/or treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, nasosinusitis, hidradenitis suppurativa, or cancer.

The present disclosure also relates to a method for preventing and/or treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, nasosinusitis, hidradenitis suppurativa, or cancer, comprising administering a therapeutically effective amount of the above crystal form A, crystal form B, crystal form C, crystal form D or crystal form E or the above composition, to a subject in need thereof.

The present disclosure also relates to the above crystal form A, crystal form B, crystal form C, crystal form D or crystal form E or the above composition, for use in preventing and/or treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, nasosinusitis, hidradenitis suppurativa, or cancer.

The term "2*θ* or 2*θ* angle" in the present disclosure refers to a diffraction angle, *θ* being the Bragg angle, and the unit of which is ° or degree. The error range of 2*θ* of each characteristic peak is ±0.20 (including the case in which a number with more than one decimal is rounded), and specifically, -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, and 0.20.

The term "excipient" in the present disclosure includes, but is not limited to, any adjuvant, carrier, glidant, sweetener, diluent, preservative, dye/colorant, flavor enhancer, surfactant, wetting agent, dispersant, suspending agent, stabilizer, isotonic agent or emulsifier that has been approved by the U.S. Food and Drug Administration and is acceptable for use in humans or livestock animals.

The starting material used in the method for preparing the crystal form of the present disclosure can be a compound in any form, and the specific form includes, but is not limited to, amorphous form, arbitrary crystal form, hydrate, solvate and the like.

The term "differential scanning calorimetry or DSC" in the present disclosure means to measure the temperature difference and heat flow difference between the sample and the reference during the heating or constant temperature process of the sample, to characterize all physical and chemical changes associated with the thermal effect, and to obtain phase change information of the sample.

In the present disclosure, the values, such as those related to the determination and calculation of substance contents, inevitably have a degree of error. In general, ±10% belongs to a reasonable range of error. There is a degree of error depending on the context in which it is used, and the error is no more than ±10% and may be ±9%, ±8%, ±7%, ±6%, ±5%, ±4%, ±3%, ±2% or ±1%, preferably ±5%.

### DESCRIPTION OF THE DRAWINGS

Figure 1 is the X-ray powder diffraction (XRPD) pattern of the crystal form A;
Figure 2 is the DSC spectrum of the crystal form A;
Figure 3 is the thermogravimetric analysis (TGA) spectrum of the crystal form A;
Figure 4 is the dynamic vapor sorption (DVS) plot of the crystal form A;
Figure 5 is the XRPD patterns of the crystal form B before and after DVS, in which the upper XRPD pattern is one after DVS and the lower XRPD pattern is one before DVS;
Figure 6 is the XRPD pattern of the crystal form B;
Figure 7 is the DSC spectrum of the crystal form B;
Figure 8 is the TGA spectrum of the crystal form B;
Figure 9 is the DVS plot of the crystal form B;
Figure 10 is the XRPD patterns of the crystal form B before and after DVS, in which the upper XRPD pattern is one after DVS and the lower XRPD pattern is one before DVS;
Figure 11 is the XRPD pattern of the crystal form C;
Figure 12 is the DSC spectrum of the crystal form C;
Figure 13 is the TGA spectrum of the crystal form C;
Figure 14 is the DVS plot of the crystal form C;
Figure 15 is the XRPD patterns of the crystal form C before and after DVS, in which the upper XRPD pattern is one after DVS and the lower XRPD pattern is one before DVS;
Figure 16 is the XRPD pattern of the crystal form D;
Figure 17 is the DSC spectrum of the crystal form D;
Figure 18 is the TGA spectrum of the crystal form D;
Figure 19 is the DVS plot of the crystal form D;
Figure 20 is the XRPD patterns of the crystal form D before and after DVS, in which the upper XRPD pattern is one before DVS and the lower XRPD pattern is one after DVS;
Figure 21 is the XRPD pattern of the crystal form E;
Figure 22 is the DSC spectrum of the crystal form E;
Figure 23 is the TGA spectrum of the crystal form E.

### DETAILED DESCRIPTION OF THE INVENTION

The present disclosure will be explained in more detail through examples or experimental examples below. The examples or experimental examples of the present disclosure are merely intended to describe the technical solutions of the present disclosure, and should not be considered as limiting the spirit and scope of the present disclosure.

The abbreviations used in the present disclosure are explained as follows:
- XRPD: X-ray powder diffraction
- DSC: Differential scanning calorimetry
- TGA: Thermogravimetric analysis
- DVS: Dynamic vapor sorption
- ¹H-NMR: Liquid nuclear magnetic resonance hydrogen spectroscopy
- DMF: N,N-dimethylformamide
- MEK: Butanone
- MTBE: Methyl tert-butyl ether
- THF: Tetrahydrofuran
- IPA: Isopropanol
- ACN: Acetonitrile
- MeOH: Methanol
- EOH: Ethanol
- ACT: Acetone
- EA: Ethyl acetate
- PA: Phosphoric acid
- TA: Tartaric acid
- HBr: Hydrobromic acid
- HCl: Hydrochloric acid
Test conditions for the instruments used in experiments of the present disclosure:
1. X-ray Powder Diffraction, XRPD
   Instrument type: Malver Panalytical Aeris X-ray powder diffractometer
   Ray: monochromatic Cu-Kα ray (λ=1.54188)
   Scanning mode: θ/2θ, Scanning range (2θ range) : 3.5-50°
   Voltage: 40 kV, Electric current: 15 mA
2. Differential Scanning Calorimetry, DSC
   Instrument type: TA DSC250
   Purge gas: Nitrogen; Nitrogen purge rate: 50 mL/min
   Heating rate: 10°C/min
   Temperature range: 25°C-300°C
3. Thermogravimetric Analysis, TGA
   Instrument type: TA TGA550
   Purge gas: Nitrogen; Nitrogen purge rate: 20 ml/min
   Heating rate: 10°C/min
   Temperature range: 30°C-350°C
4. Dynamic vapor sorption (DVS)
   Using SMS Intrinsic PLUS, the detection is performed at 25°C, with humidity change of 50% -0% -90% with a step size of 10%, in which the criteria for judging is that the mass change dM/dT for each gradient is less than 0.002%, TMAX is 360 min, and two cycles are performed.
5. High performance liquid chromatography (HPLC) described in the stability test of the crystal forms of the present disclosure is determined on Agilent 1260 Infinity II.

In the content detection method by the high performance liquid chromatography described in the present disclosure, HPLC conditions are as follows: chromatographic column, Agilent Eclipse Plus C18 4.6mm*150mm,3.5µm; mobile phase, A - 10 mmol/L solution of sodium dihydrogen phosphate (pH 8.0), and B - ACN; flow rate, 1.0 ml/min; wavelength, 210 nm.

In the related substances detection method by the high performance liquid chromatography described in Example 8 of the present disclosure, HPLC conditions are as follows: chromatographic column, Agilent Eclipse Plus C18 4.6mm*150mm,3.5µm; mobile phase, A - 10 mmol/L solution of ammonium acetate, and B - ACN; flow rate, 1.0 ml/min; wavelength, 210 nm.

In the detection method of isomers of formula I by the high performance liquid chromatography described in Examples of the present disclosure, conditions are as follows: chromatographic column, CHIRALPAK IG-3 4.6mm*250mm 3µm or CHIRALPAK IG-3 4.0mm*10mm 3µm; mobile phase, A - n-hexane, B - anhydrous ethanol, and n-hexane : anhydrous ethanol = 25:75; flow rate, 1.0 ml/min; wavelength, 254 nm.

### Example 1: Preparation of the compound of formula I: (S)-N-((S)-1-cyano-2-(8-cyano-2-fluoro-6H-benzo[c]chromen-3-yl)ethyl)-1,4-oxazepane-2-carboxamide

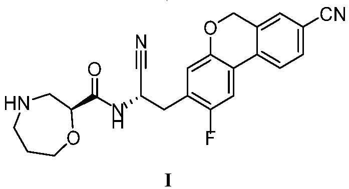

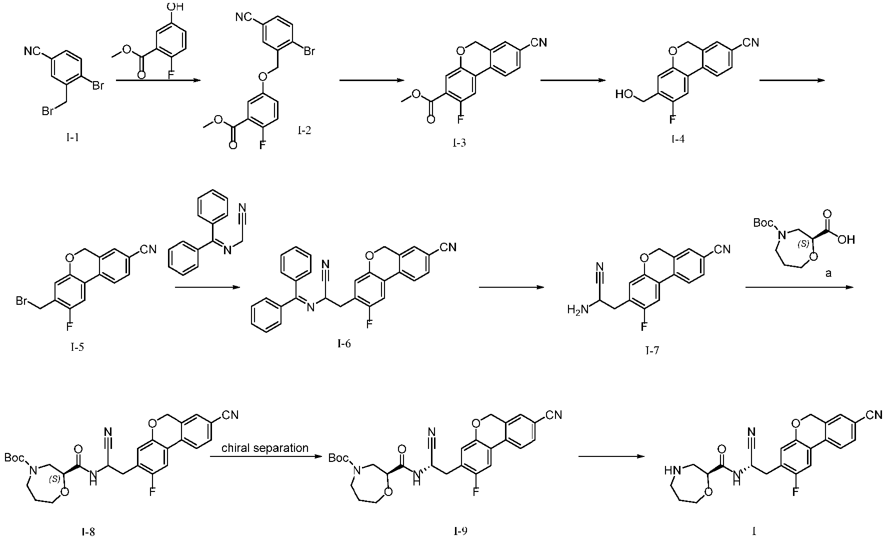

### Synthesis of Compound I-2

Methyl 2-fluoro-5-hydroxybenzoate (4.30 g, 25.29 mmol), 4-bromo-3-(bromomethyl)benzonitrile (Compound **I-1)** (7.72 g, 25.30 mmol) and potassium carbonate (6.99 g, 50.58 mmol) were dissolved in *N,N*-dimethylformamide (50 mL) at room temperature, and the reaction mixture was heated to 40°C and stirred for 12 hours. Water (300 mL) was added thereto, and the mixture was extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain Compound **I-2.** ¹H NMR (400 MHz, CDCl₃) δ 7.87 (d, 1H), 7.73 (d, 1H), 7.55-7.48 (m, 2H), 7.18-7.09 (m, 2H), 5.11 (s, 2H), 3.95 (s, 3H).

### Synthesis of Compound I-3

Compound **I-2** (2.90 g, 6.37 mmol), palladium acetate (0.14 g, 0.64 mmol), potassium carbonate (1.76 g, 12.73 mmol) and tricyclohexylphosphine tetrafluoroborate (0.23 g, 0.64 mmol) were dissolved in *N,N-*dimethylformamide (30 mL) at room temperature under nitrogen atmosphere, and the reaction mixture was heated to 120°C and stirred for 1.5 hours. After the reaction was complete, the resulting mixture was cooled to room temperature, followed by the addition of water (200 mL), and extracted with ethyl acetate (200 mL×2). The organic phases were combined, washed with saturated brine (200 mL), dried over anhydrous sodium sulfate and filtered. The filtrate was concentrated under reduced pressure, and the resulting residue was purified by column chromatography (SiO₂, petroleum ether/ethyl acetate=4/1) to obtain Compound **1-3.** ¹H NMR (400 MHz, CDCl₃) δ 7.80-7.72 (m, 2H), 7.60 (d, 1H), 7.52-7.49 (m, 2H), 5.18 (s, 2H), 3.98 (s, 3H).

### Synthesis of Compound I-4

Compound **I-3** (1.20 g, 3.81 mmol) and lithium borohydride (0.25 g, 11.47 mmol) were dissolved in tetrahydrofuran (25 mL) at room temperature, and the reaction mixture was heated to 55°C and stirred for 2 hours. After the reaction was complete, the reaction mixture was cooled to room temperature, followed by the addition of water (200 mL), and extracted with ethyl acetate (200 mL×2). The organic phases were combined, washed with saturated brine (200 mL), dried over anhydrous sodium sulfate and filtered. The filtrate was concentrated under reduced pressure, and the resulting residue was purified by column chromatography (SiO₂, petroleum ether/ethyl acetate=9/1) to obtain Compound **I-4.** ¹H NMR (400 MHz, CDCl₃) δ 7.67 (s, 2H), 7.47 (s, 1H), 7.38 (d, 1H), 7.12 (d, 1H), 5.11 (s, 2H), 4.77 (s, 2H).

### Synthesis of Compound I-5

Compound **I-3** (450 mg, 1.59 mmol) was dissolved in dichloromethane (15 mL) and then phosphorus tribromide (520 mg, 1.92 mmol) was added dropwise thereto at room temperature, and the reaction mixture was stirred for 20 minutes at room temperature. After the reaction was complete, water (100 mL) was added thereto, and the resulting mixture was extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain Compound **I-5.** ¹H NMR (300 MHz, CDCl₃) δ 7.73 (s, 2H), 7.52 (s, 1H), 7.45 (d, 1H), 7.10 (d, 1H), 5.17 (s, 2H), 4.53 (s, 2H).

### Synthesis of Compound I-6

N-(diphenylmethylene)aminoacetonitrile (250 mg, 1.14 mmol), Compound **I-5** (470 mg, 1.26 mmol), benzyltrimethylammonium chloride (22 mg, 0.12 mmol) and sodium hydroxide (91 mg, 2.3 mmol) were dissolved in a mixed solvent of dichloromethane (6 mL) and water (6 mL) at room temperature, and the reaction mixture was heated to 35°C and stirred for 24 hours. Water (30 mL) was added thereto, and the resulting mixture was extracted with dichloromethane (30 mL×2). The organic phases were combined, washed with saturated brine (20 mL), dried over anhydrous sodium sulfate and filtered. The filtrate was concentrated under reduced pressure, and the resulting residue was purified by column chromatography (SiO₂, petroleum ether/ethyl acetate=4/1) to obtain Compound **1-6.** MS-ESI: *m*/*z* 458.4 [M+1]⁺.

### Synthesis of Compound I-7

Compound **I-6** (520 mg, 0.90 mmol) was dissolved in tetrahydrofuran (10 mL) and then 1 M aqueous hydrochloric acid solution (4 mL) was added dropwise thereto at room temperature, and the reaction mixture was stirred for 1 hour at room temperature. After the reaction was complete, saturated aqueous sodium bicarbonate solution (30 mL) was added thereto, and the resulting mixture was extracted with ethyl acetate (30 mL×3). The organic phases were combined, washed with saturated brine (30 mL), dried over anhydrous sodium sulfate and filtered. The filtrate was concentrated under reduced pressure, and the resulting residue was purified by column chromatography (SiO₂, petroleum ether/ethyl acetate=2/1) to obtain Compound **I-7**. MS-ESI: *m*/*z* 293.9 [M+1]⁺.

### Synthesis of Compound I-8

Compound **I-7** (220 mg, 0.68 mmol), Compound **a** (170 mg, 0.69 mmol), *N,N,N',N'-*tetramethyl-O-(7-azabenzotriazol-1-yl)uronium hexafluorophosphate (360 mg, 0.95 mmol) and *N,N*-diisopropylethylamine (250 mg, 1.93 mmol) were dissolved in *N,N-*dimethylformamide (5 mL) at room temperature, and the reaction mixture was stirred for 3 hours at room temperature. After the reaction was complete, the resulting mixture was purified by preparative liquid chromatography (C18, acetonitrile/water system) to obtain Compound **I-8.** MS-ESI: *m*/*z* 465.1 [M-56+1]⁺.

### Synthesis of Compound I-9

Compound **I-8** (280 mg, 0.51 mmol) was subjected to chiral resolution (column: chiralpak IE, 250*25 mm, 5 µm; mobile phase: n-hexane, ethanol; gradient: n-hexane phase 30%; flow rate: 15 mL/min; column temperature: 30°C) to obtain Compound **I-9** (two diastereomer peaks in total, Compound **I-9** being the first elution peak). ¹H NMR (300 MHz, DMSO-*d*₆) δ 8.89 (d, 1H), 8.08 (d, 1H), 7.94-7.89 (m, 2H), 7.83 (s, 1H), 7.07 (d, 1H), 5.24-5.14 (m, 2H), 5.09-5.07 (m, 1H), 4.15-4.12 (m, 1H), 3.99-3.87 (m, 2H), 3.63-3.56 (m, 2H), 3.29-3.02 (m, 4H), 1.89-1.79 (m, 2H), 1.41-1.36 (m, 9H).

### Synthesis of Compound I

Compound **I-9** (85 mg, 0.16 mmol) was dissolved in formic acid (1 mL) at room temperature, and the reaction mixture was heated to 40°C and stirred for 1 hours. After the reaction was complete, the resulting mixture was purified by preparative liquid chromatography (C18, ammonium bicarbonate/acetonitrile/water system) to obtain Compound **I.** The resulting compound was determined as amorphous by XRPD.

MS-ESI: *m*/*z* 421.2 [M+1]⁺. ¹H NMR (400 MHz, DMSO-*d*₆) δ 8.69 (d, 1H), 8.05 (d, 1H), 7.89-7.86 (m, 2H), 7.80 (s, 1H), 7.03 (d, 1H), 5.20-5.12 (m, 2H), 5.09-5.02 (m, 1H), 4.00-3.97 (m, 1H), 3.88-3.83 (m, 1H), 3.77-3.68 (m, 1H), 3.25-3.14 (m, 2H), 3.05-2.98 (m, 1H), 2.82-2.72 (m, 1H), 2.62-2.53 (m, 2H), 1.80-1.66 (m, 2H).

### Example 2: Biological evaluation of the compound of formula I - In vitro CatC cell activity experiment

### 1. Experimental materials

| **Name** | **Brand** | **Cat No./Model** |
|---|---|---|
| U937 | ATCC | CRL-1593.2 |
| H-Gly-Phe-AMC | Lai' ang | P201221-K3 |
| AZD7986 (alias: INS 1007) | MedChemExpress | HY-101056 |
| OptiPlate^{™}-384 F black-bottom analysis plate | PerkinElmer | 6007279 |
| RPMI1640 culture medium +10%FBS | Gbico | |

### 2. Experimental steps

Complete culture medium RPMI 1640+10% FBS was formulated and mixed well. The U937 cell line was resuscitated and passaged for about two passages, and the cell strains with good growth status were selected. The cell suspension was transferred into a centrifuge tube, and centrifuged at 800 to 1000 rpm for 3 to 5 minutes. The supernatant was discarded. An appropriate volume of culture medium was added to the centrifuge tube, which was gently pipetted to resuspend the cells evenly. The cells were counted using a Vi-Cell XR cell counter. The cell suspension was adjusted to an appropriate concentration. The cell suspension was added into a 384-well plate, at 25000 µL/well. The compound was formulated into a 10 mM solution with DMSO, diluted with DMSO to obtain a 1 mM solution, and subjected to a semilogarithmic dilution by HPD300 with DMSO to obtain 10 concentrations. Gly-Phe-AFC was formulated into a 35 mM solution with DMSO and divided. Gly-Phe-AFC was formulated to 1.75 mM with serum-free culture medium. After adding the compounds and incubating for 1 hour in an incubator, the substrate-AFC was added (12.5 µL was added to the plate). The plate was incubated for 30 minutes, followed by measurement. The plate was placed and read in EnSpire, and the fluorescence values were recorded at Ex 400 nm and Em 505 nm. The inhibition rate was calculated according to the following formula: inhibition rate (%) = (1 - (RFU compound - RFU blank)/(RFU DMSO - RFU blank)) × 100%. XLFit was used to draw the drug efficacy inhibition rate curve and calculate the IC₅₀ value. 4-parameter model [fit = (A + ((B - A)/ (1 + ((C/x) ^D))))] was used.

The *in vitro* inhibition of the compound of formula I of the present disclosure on CatC cell activity was determined through the above test, and the determined IC₅₀ value was 6.1 nM, indicating that the inhibitory effect on CatC cell activity was significant.

### Example 3: Preparation of crystal form A of the compound of formula I

10 mg of the compound of formula I was added to 0.2 ml of the solvent, kept in a triturating state in the solvent, and stirred for one week at room temperature (about 25°C) or 50°C and then filtrated. The resulting solid was determined as free crystal form A by XRPD, as shown in Table 1 below.

**Table 1. Solvents selected for the preparation of crystal form A through trituration crystallization and the determination results by XRPD**

| Solvent (molar ratio of water in the mixed solvent) | Crystal form (stirring at room temperature) | Crystal form (stirring at 50°C) |
|---|---|---|
| Water | A | A |
| MeOH | A | A |
| EOH | A | A |
| 0.14 (Water/ MeOH) | A | A |
| 0.26 (Water/ MeOH) | A | A |
| 0.37 (Water/ MeOH) | A | A |
| 0.47 (Water/ MeOH) | A | A |
| 0.57 (Water/ MeOH) | A | A |
| 0.66 (Water/ MeOH) | A | A |
| 0.74 (Water/ MeOH) | A | A |
| 0.82 (Water/ MeOH) | A | A |
| 0.90 (Water/ MeOH) | A | A |

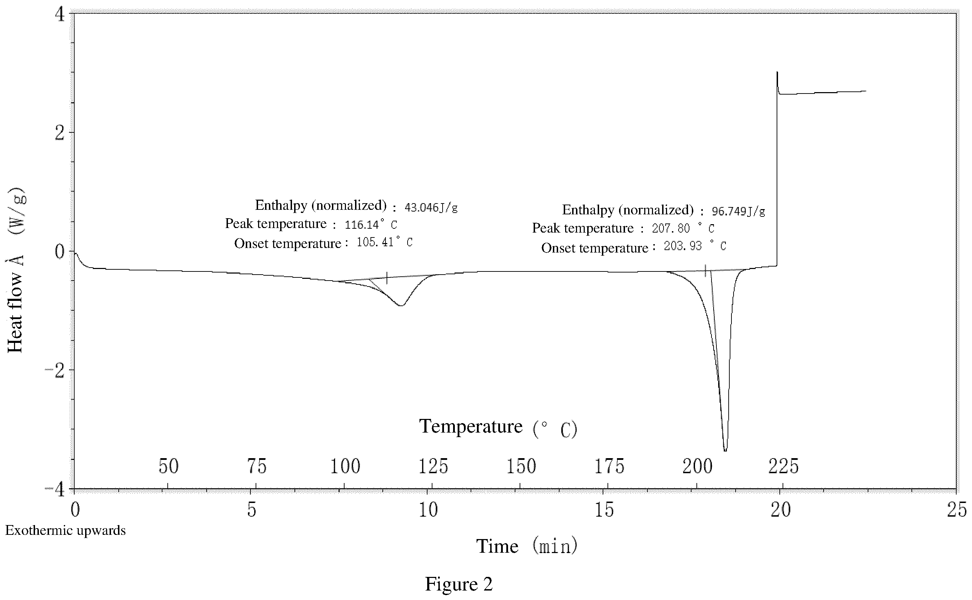

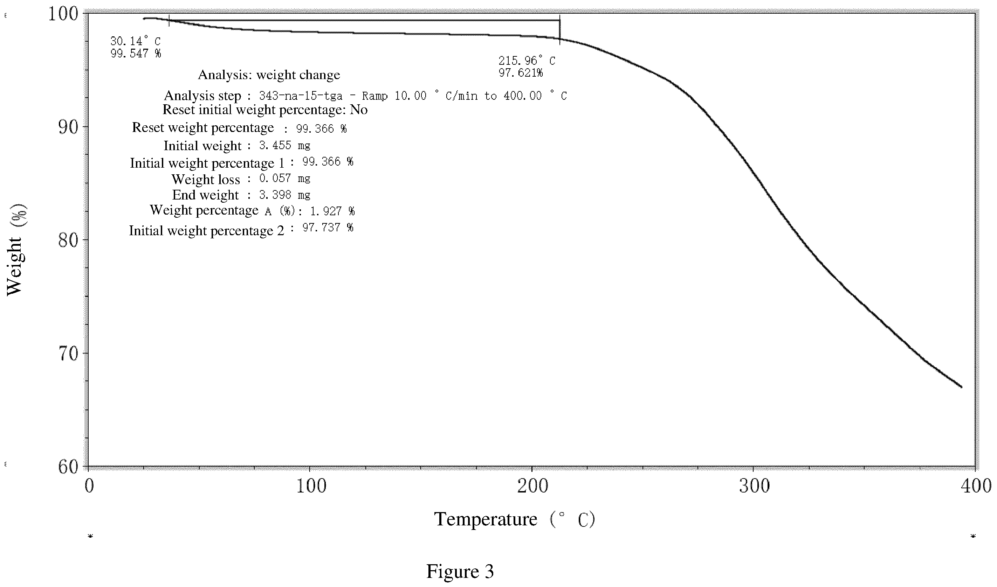

The product was detected by the X-ray powder diffraction and defined as crystal form A. The XRPD pattern is shown in Figure 1, and the positions of the characteristic peaks are shown in Table 2. The DSC spectrum, as shown in Figure 2, shows endothermic peaks at 116°C and 208°C. The TGA spectrum, as shown in Figure 3, shows a weight loss of 2% before 100°C and a rapid weight loss at 200°C or higher.

**Table 2. Data of XRPD characteristic diffraction peaks of crystal form A**

| Peak No. | 2*θ*[° or degree] | d[Å] | Relative strength |
|---|---|---|---|
| 1 | 8.910 | 9.925 | 100.00 |
| 2 | 10.273 | 8.611 | 73.03 |
| 3 | 11.964 | 7.397 | 7.56 |
| 4 | 13.730 | 6.450 | 25.73 |
| 5 | 15.650 | 5.663 | 80.05 |
| 6 | 17.869 | 4.964 | 26.76 |
| 7 | 18.617 | 4.766 | 47.49 |
| 8 | 19.526 | 4.546 | 23.58 |
| 9 | 20.785 | 4.274 | 20.35 |
| 10 | 22.193 | 4.006 | 25.34 |
| 11 | 23.452 | 3.793 | 41.77 |
| 12 | 24.347 | 3.656 | 25.93 |
| 13 | 25.049 | 3.555 | 28.58 |
| 14 | 26.590 | 3.352 | 22.66 |
| 15 | 28.272 | 3.157 | 17.52 |
| 16 | 29.980 | 2.981 | 9.41 |
| 17 | 33.168 | 2.701 | 6.64 |
| 18 | 39.680 | 2.272 | 4.91 |
| 19 | 43.444 | 2.083 | 1.45 |

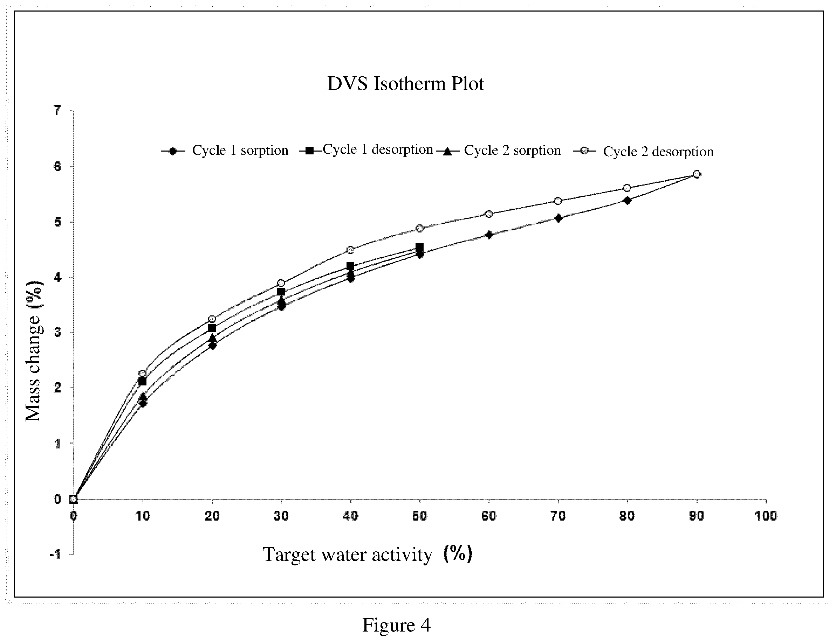

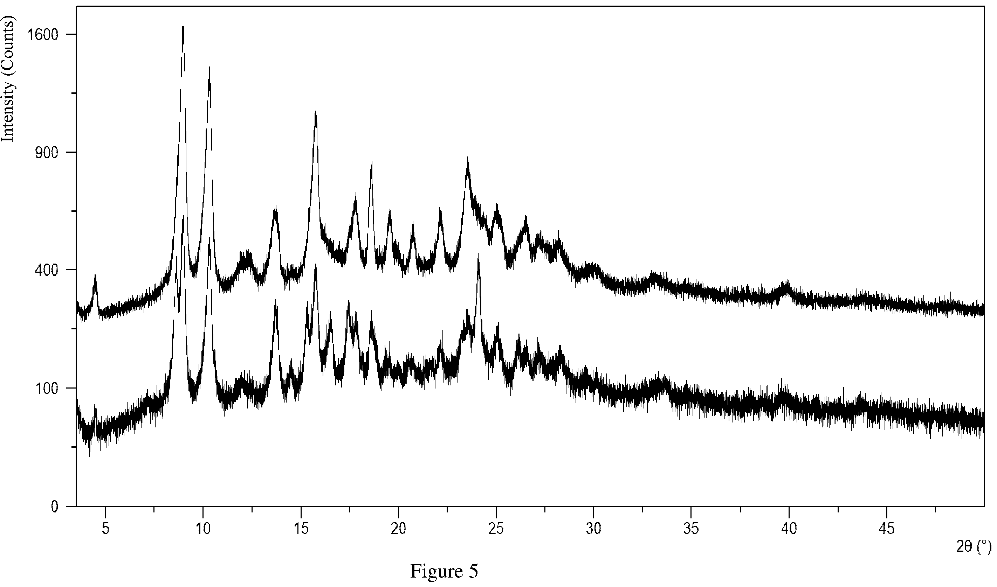

The DVS detection, as shown in Figure 4, shows that the weight gain by hygroscopicity is approximately 5.39% under accelerated experimental conditions (i.e., 80% RH). During the humidity change of 0% -90% RH, the desorption process of the sample coincides with the adsorption process thereof. After DVS detection, the crystal form was redetermined, and the XRPD pattern is shown in Figure 5, showing that the crystal form did not change before and after DVS detection.

### Example 4: Preparation of crystal form B of the compound of formula I

About 100 mg of Compound **I** was added to 1 ml of the solvent, kept in a triturating state in the solvent, and stirred for seven days at room temperature (about 25°C) or 50°C and then filtrated. The resulting solid was determined as free crystal form B by XRPD, as shown in Table 3 below.

**Table 3. Solvents selected for the preparation of crystal form B through trituration crystallization and the determination results by XRPD**

| Solvent | Crystal form (stirring at room temperature) | Crystal form (stirring at 50°C) |
|---|---|---|
| Nitromethane | B | B |
| Acetonitrile | B | B |

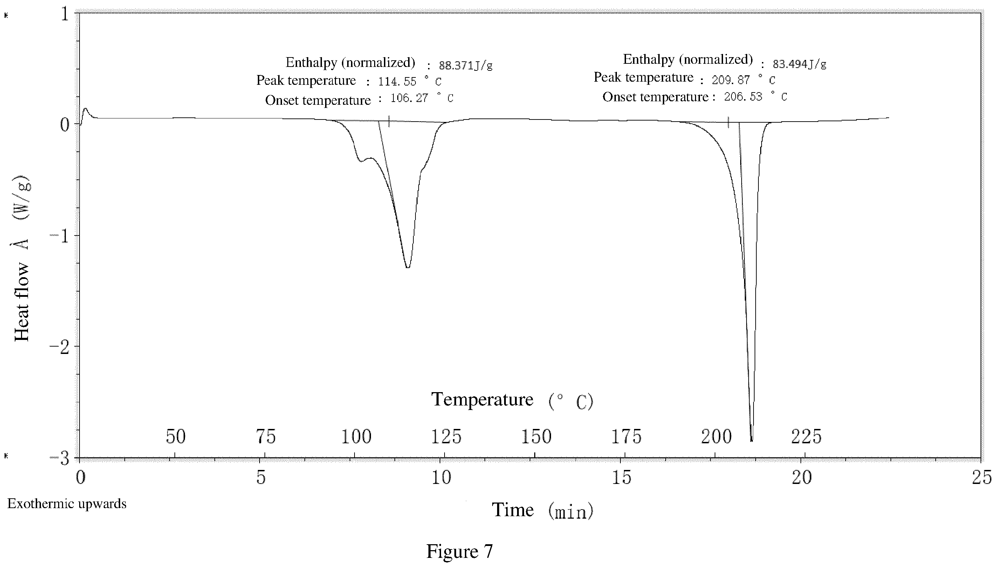

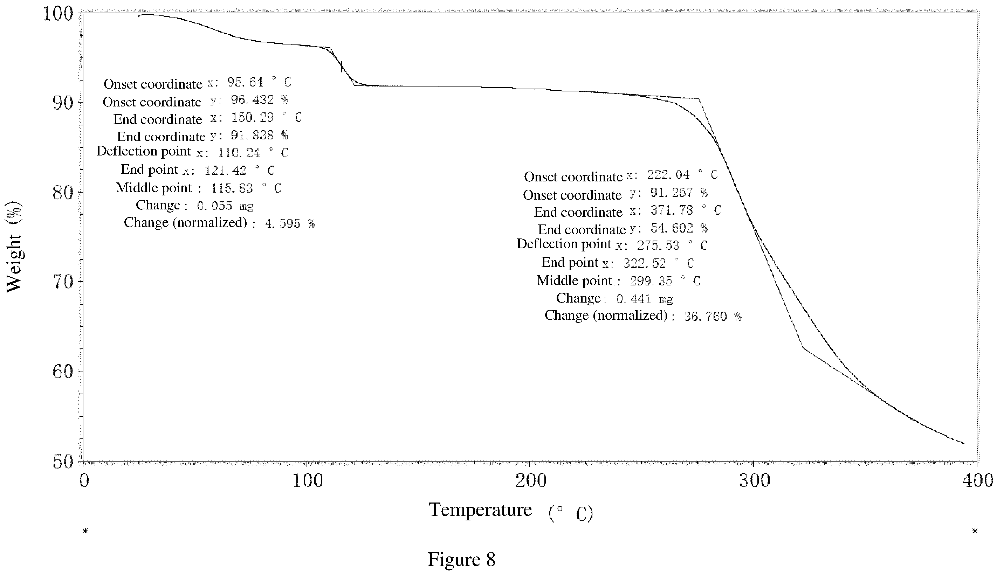

The product was detected by the X-ray powder diffraction and defined as crystal form B. The XRPD pattern is shown in Figure 6, and the positions of the characteristic peaks are shown in Table 4. The DSC spectrum, as shown in Figure 7, shows endothermic peaks at 114.55°C and 209.87°C. The TGA spectrum is shown in Figure 8.

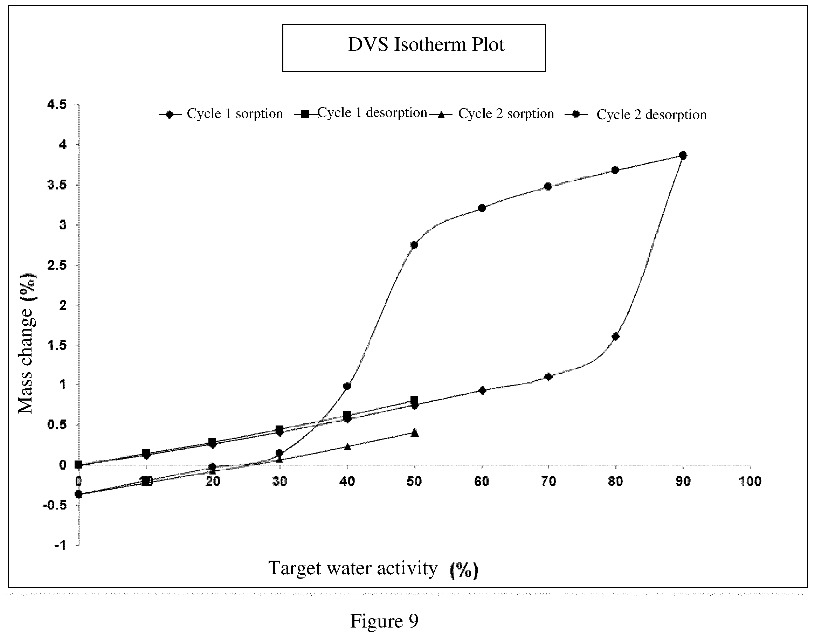

The DVS detection, as shown in Figure 9, shows that: under normal storage conditions (i.e., 25°C, 60% RH), the weight gain by hygroscopicity of the sample is approximately 0.93%; under accelerated experimental conditions (i.e., 80% RH), the weight gain by hygroscopicity is approximately 1.60%; under extreme conditions (i.e., 90% RH), the weight gain by hygroscopicity is approximately 3.87%. After DVS detection, the crystal form was redetermined, and the XRPD pattern is shown in Figure 10, showing that the crystal form did not change before and after DVS detection.

**Table 4. Data of XRPD characteristic diffraction peaks of crystal form B**

| Peak No. | 2*θ*[° or degree] | d[Å] | Relative strength |
|---|---|---|---|
| 1 | 6.770 | 13.05712 | 84.48 |
| 2 | 11.201 | 7.89951 | 100 |
| 3 | 12.989 | 6.81591 | 42.48 |
| 4 | 14.931 | 5.93364 | 72.67 |
| 5 | 15.851 | 5.59107 | 8.2 |
| 6 | 17.228 | 5.14731 | 7.18 |
| 7 | 18.719 | 4.74046 | 2.32 |
| 8 | 20.817 | 4.26715 | 37.03 |
| 9 | 22.313 | 3.98436 | 5.88 |
| 10 | 22.773 | 3.9049 | 6.63 |
| 11 | 24.381 | 3.65097 | 3.49 |
| 12 | 25.710 | 3.46512 | 35.54 |
| 13 | 27.305 | 3.26626 | 4.74 |
| 14 | 28.192 | 3.16542 | 7.34 |
| 15 | 31.066 | 2.87885 | 10.99 |
| 16 | 32.046 | 2.79305 | 14.49 |
| 17 | 32.913 | 2.72143 | 10.28 |

### Example 5: Preparation of crystal form C of the compound of formula I

About 100 mg of Compound **I** was added to 1 ml of the solvent, kept in a triturating state in the solvent, and stirred for seven days at room temperature (about 25°C) or 50°C and then filtrated. The resulting solid was determined as crystal form C by XRPD, as shown in Table 5 below.

**Table 5. Solvents selected for the preparation of crystal form C through trituration crystallization and the determination results by XRPD**

| Solvent | Crystal form (stirring at room temperature) | Crystal form (stirring at 50°C) |
|---|---|---|
| DMF | C | C |
| Acetone | C | C |
| MEK | C | C |
| THF | C | / |
| 1,2-Dimethylethane | C | C |

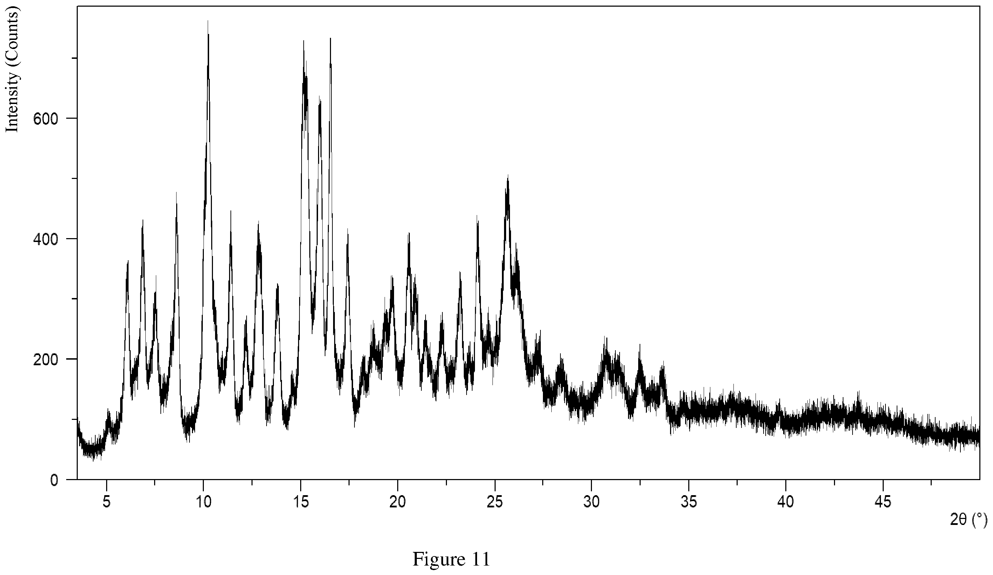

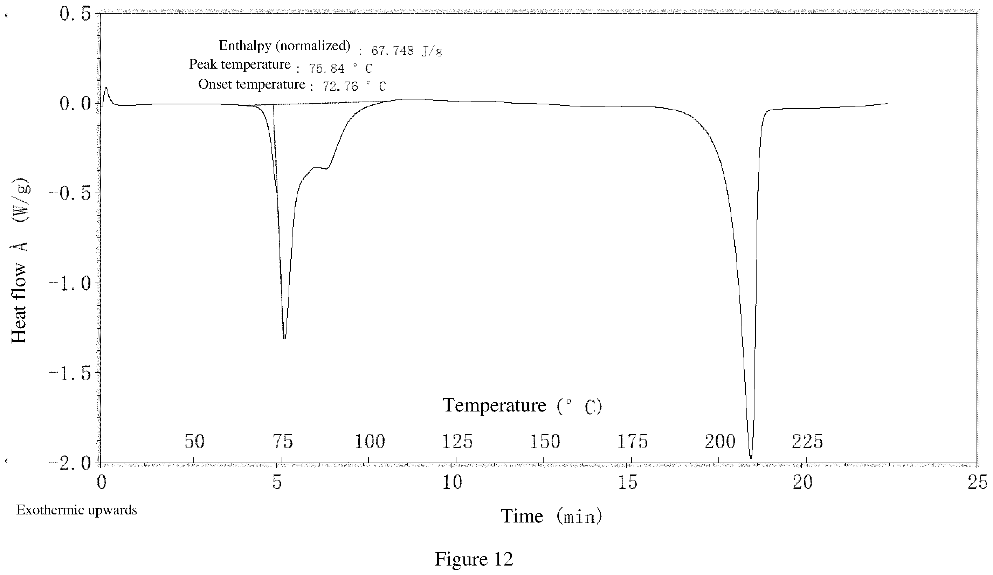

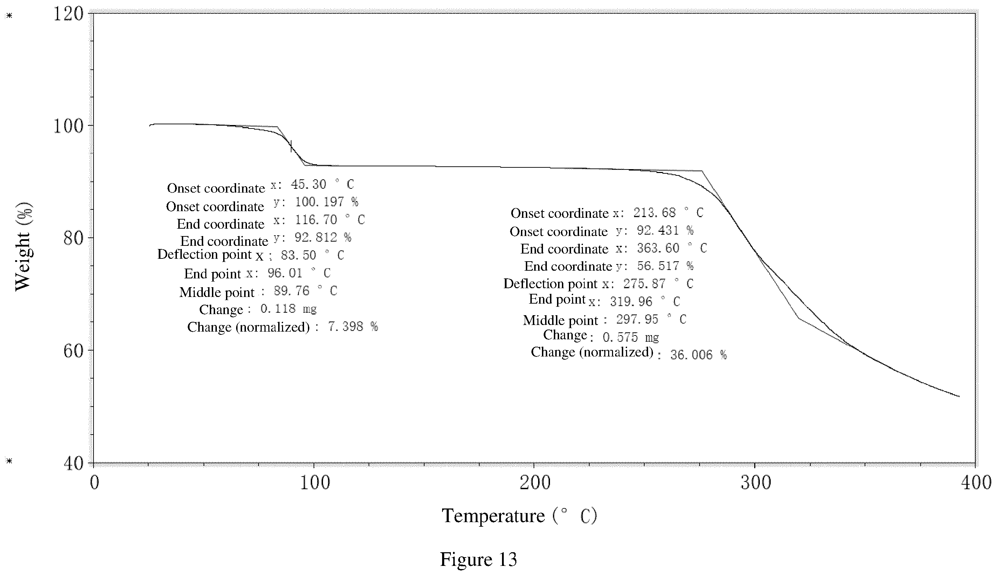

The product was detected by the X-ray powder diffraction and defined as crystal form C. The XRPD pattern is shown in Figure 11, and the positions of the characteristic peaks are shown in Table 6. The DSC spectrum, as shown in Figure 12, shows endothermic peaks at 75.84°C and 208.87°C. The TGA spectrum is shown in Figure 13.

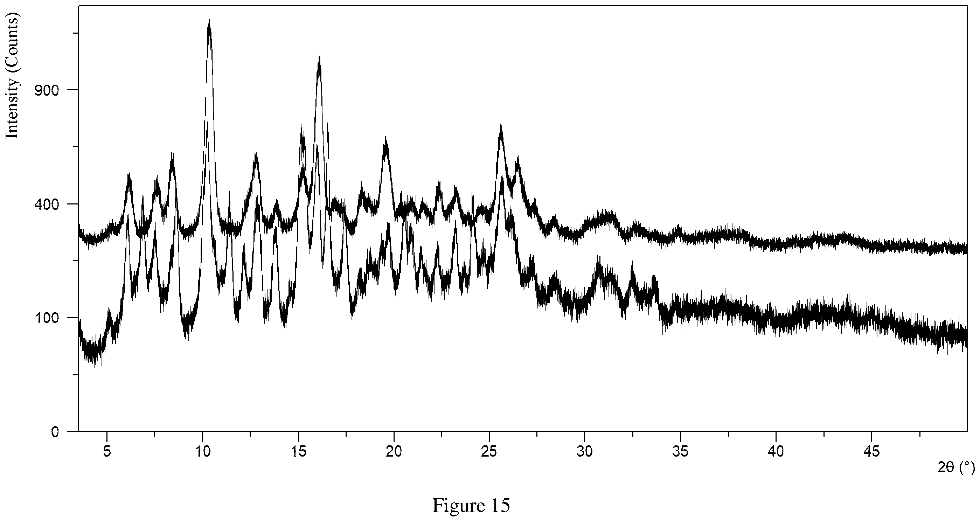

The DVS detection, as shown in Figure 14, shows that: under normal storage conditions (i.e., 25°C, 60% RH), the weight gain by hygroscopicity of the sample is approximately 0.65%; under accelerated experimental conditions (i.e., 80% RH), the weight gain by hygroscopicity is approximately 0.89%; under extreme conditions (i.e., 90% RH), the weight gain by hygroscopicity is approximately 1.16%. After DVS detection, the crystal form was redetermined, and the XRPD pattern is shown in of Figure 15, showing that the crystal form did not change before and after DVS detection.

**Table 6. Data of XRPD characteristic diffraction peaks of crystal form C**

| Peak No. | 2*θ*[° or degree] | d[Å] | Relative strength |
|---|---|---|---|
| 1 | 6.012 | 14.70084 | 19.6 |
| 2 | 7.385 | 11.97073 | 22.58 |
| 3 | 8.275 | 10.68502 | 15.09 |
| 4 | 10.171 | 8.69695 | 100 |
| 5 | 11.275 | 7.84802 | 3.88 |
| 6 | 12.687 | 6.97776 | 30.57 |
| 7 | 13.720 | 6.4542 | 7.67 |
| 8 | 15.120 | 5.85993 | 18.42 |
| 9 | 15.902 | 5.57319 | 83.7 |
| 10 | 16.842 | 5.26433 | 6.3 |
| 11 | 19.645 | 4.51902 | 19.8 |
| 12 | 20.739 | 4.28315 | 6.52 |
| 13 | 22.230 | 3.99914 | 3.9 |
| 14 | 22.924 | 3.87954 | 2.86 |
| 15 | 24.612 | 3.6171 | 4.64 |
| 16 | 25.539 | 3.48797 | 23.05 |
| 17 | 26.382 | 3.37843 | 11.73 |
| 18 | 27.272 | 3.27007 | 5.45 |
| 19 | 28.319 | 3.15158 | 2.61 |
| 20 | 30.887 | 2.89514 | 6.84 |
| 21 | 32.445 | 2.75955 | 3.1 |

### Example 6: Preparation of crystal form D of the compound of formula I

About 100 mg of Compound **I** was added to 1 ml of the solvent, kept in a triturating state in the solvent, and stirred for seven days at room temperature (about 25°C) or 50°C and then filtrated. The resulting solid was determined as free crystal form D by XRPD, as shown in Table 7 below.

**Table 7. Solvents selected for the preparation of crystal form D through trituration crystallization and the determination results by XRPD**

| Solvent | Crystal form (stirring at room temperature) | Crystal form (stirring at 50°C) |
|---|---|---|
| Ethyl acetate | D | D |
| 1,2-Xylene | D | D |
| Toluene | D | D |
| 1,4-Dioxane | D | D |
| Hexane | D | D |

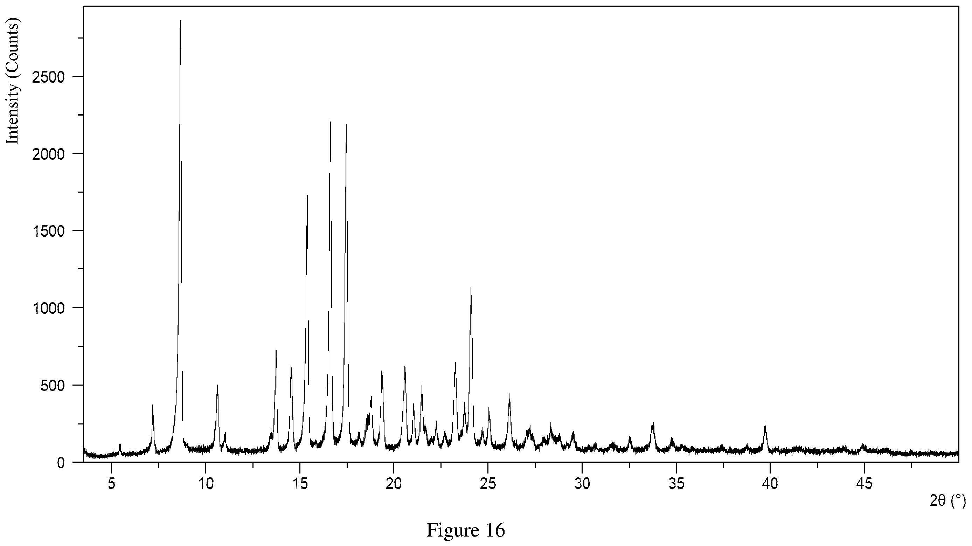

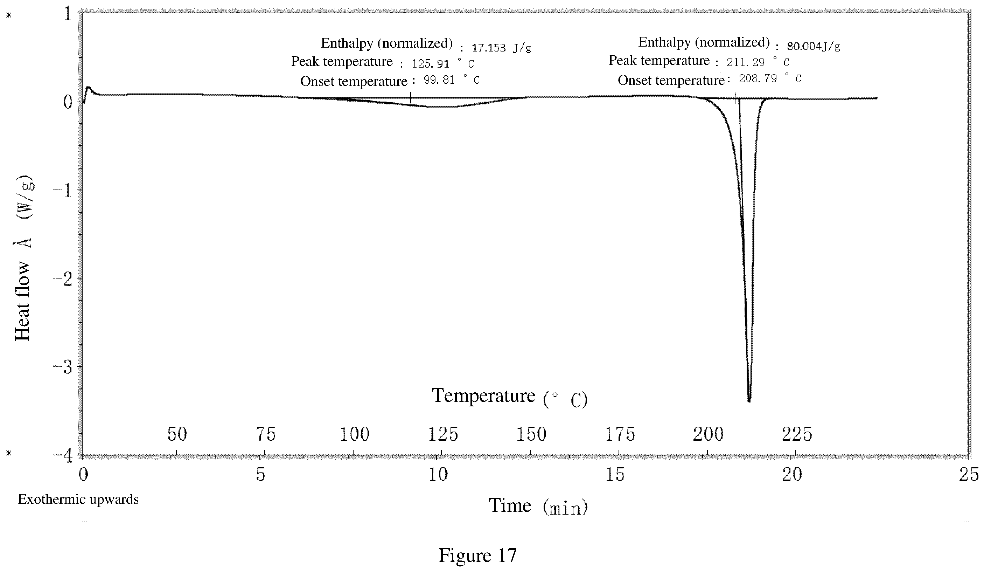

The product was detected by the X-ray powder diffraction and defined as crystal form D. The XRPD pattern is shown in Figure 16, and the positions of the characteristic peaks are shown in Table 8. The DSC spectrum, as shown in Figure 17, shows an endothermic peak at 211.29°C. The TGA spectrum is shown in Figure 18.

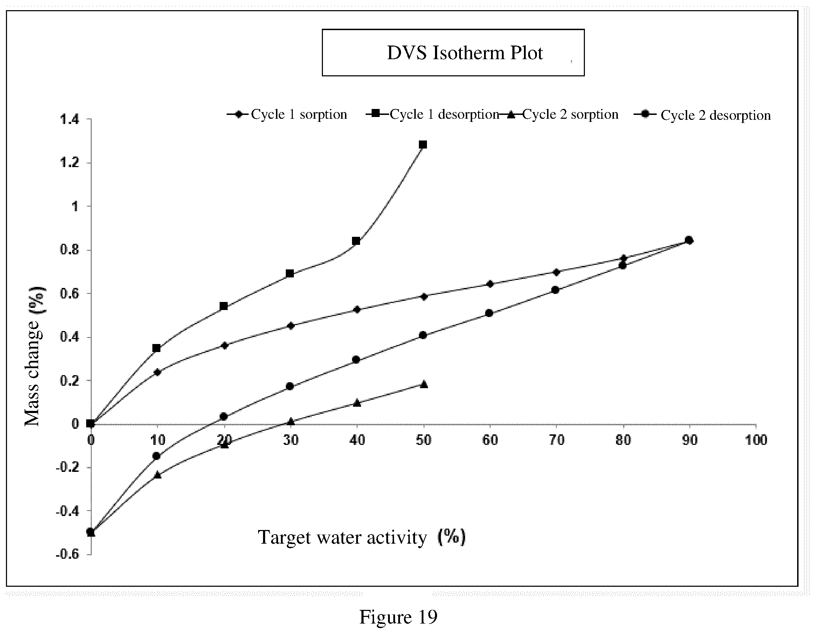

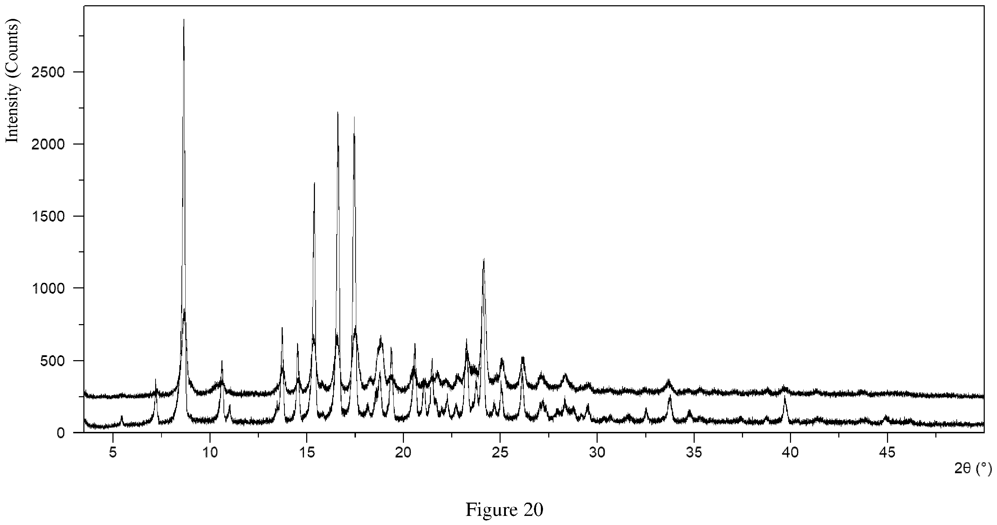

The DVS detection, as shown in Figure 19, shows that: under normal storage conditions (i.e., 25°C, 60% RH), the weight gain by hygroscopicity of the sample is approximately 0.64%; under accelerated experimental conditions (i.e., 80% RH), the weight gain by hygroscopicity is approximately 0.76%; under extreme conditions (i.e., 90% RH), the weight gain by hygroscopicity is approximately 0.84%. After DVS detection, the crystal form was redetermined, and the XRPD pattern is shown in Figure 20, showing that the crystal form did not change before and after DVS detection.

**Table 8. Data of XRPD characteristic diffraction peaks of crystal form D**

| Peak No. | 2*θ*[° or degree] | d[Å] | Relative strength |
|---|---|---|---|
| 1 | 8.477 | 10.43056 | 100 |
| 2 | 10.451 | 8.46447 | 14.51 |
| 3 | 13.569 | 6.52585 | 20.58 |
| 4 | 15.146 | 5.84995 | 58.04 |
| 5 | 16.335 | 5.4267 | 47.83 |
| 6 | 17.275 | 5.13329 | 37.82 |
| 7 | 18.775 | 4.72643 | 8.99 |
| 8 | 20.298 | 4.37519 | 13.58 |
| 9 | 23.093 | 3.8515 | 10.35 |
| 10 | 23.990 | 3.7095 | 51.81 |
| 11 | 24.921 | 3.57295 | 10.09 |
| 12 | 26.006 | 3.42633 | 12.96 |
| 13 | 27.047 | 3.29685 | 9.14 |
| 14 | 28.135 | 3.17174 | 11.27 |
| 15 | 33.461 | 2.67809 | 7.94 |

### Example 7: Preparation of crystal form E of the compound of formula I

The compound of formula I obtained in Example 1, crystal form B, crystal form C and crystal form D were dried under vacuum for 30 minutes at 120°C. The resulting solid was determined as free crystal form E by XRPD.

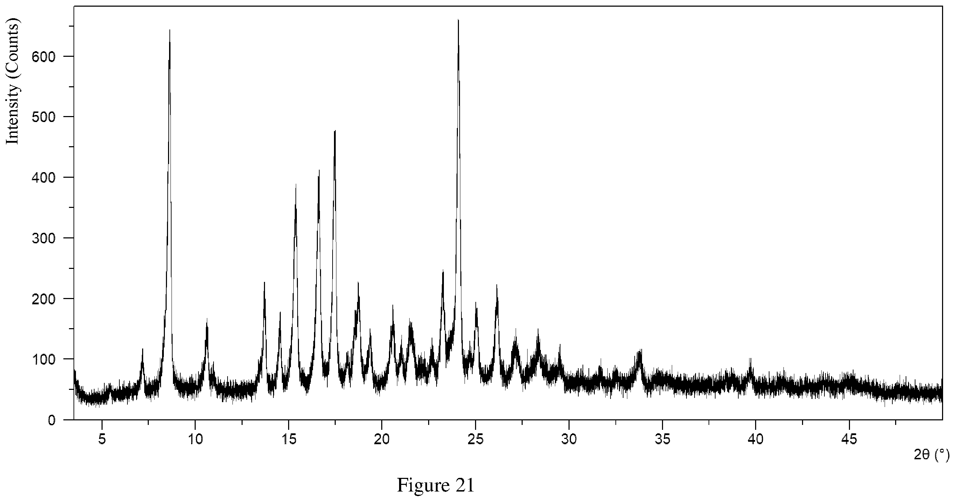

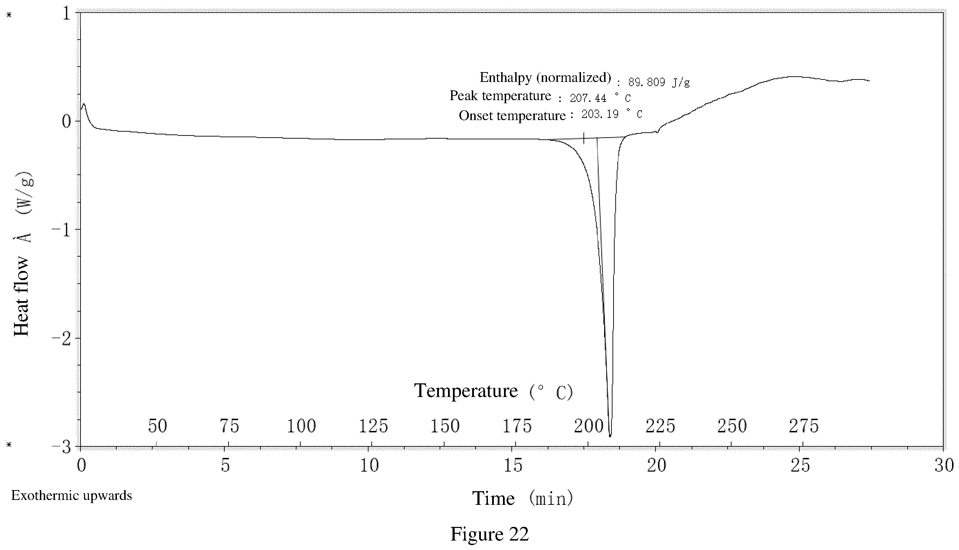

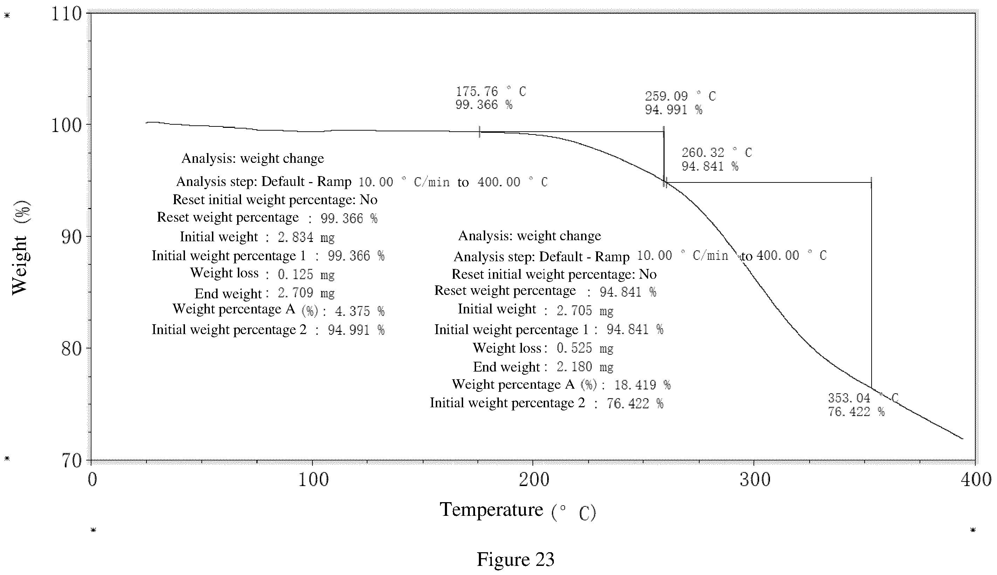

The compound of formula I obtained in Example 1, crystal form B, crystal form C and crystal form D thereof were heated to 150°C. The resulting solid was determined as free crystal form E by XRPD. The XRPD pattern is shown in Figure 21, and the positions of the characteristic peaks are shown in Table 9. The DSC spectrum, as shown in Figure 22, shows an endothermic peak at 207.44°C. The TGA spectrum is shown in Figure 23.

**Table 9. Data of XRPD characteristic diffraction peaks of crystal form E**

| Peak No. | 2*θ*[° or degree] | d[Å] | Relative strength |
|---|---|---|---|
| 1 | 5.503 | 16.0602 | 2.09 |
| 2 | 7.233 | 12.22158 | 8.04 |
| 3 | 8.685 | 10.18201 | 75.48 |
| 4 | 10.681 | 8.28267 | 13.03 |
| 5 | 13.773 | 6.42965 | 19.43 |
| 6 | 14.600 | 6.06744 | 13.63 |
| 7 | 15.433 | 5.74171 | 40.02 |
| 8 | 16.654 | 5.32321 | 43.26 |
| 9 | 17.526 | 5.06044 | 66.05 |
| 10 | 18.779 | 4.72539 | 37.77 |
| 11 | 19.393 | 4.57731 | 11.55 |
| 12 | 20.610 | 4.30961 | 13.82 |
| 13 | 21.653 | 4.10428 | 13.86 |
| 14 | 23.319 | 3.81473 | 26.14 |
| 15 | 24.151 | 3.68523 | 100 |
| 16 | 25.111 | 3.54636 | 15.21 |
| 17 | 26.192 | 3.4025 | 18.66 |
| 18 | 27.171 | 3.28201 | 7.89 |
| 19 | 28.397 | 3.14304 | 8.66 |
| 20 | 29.567 | 3.02127 | 4.63 |
| 21 | 30.685 | 2.91374 | 2.22 |
| 22 | 31.681 | 2.82439 | 2.3 |
| 23 | 32.613 | 2.74571 | 3.07 |
| 24 | 33.812 | 2.65108 | 6.93 |

### Example 8: Stability test of free base crystal form E of the compound of formula I

It is determined whether temperature and humidity have an influence on the stability of free base crystal form E of the compound of formula I.

The free base crystal form E was placed in a stabilizing chamber in the dark and subjected to influence factor tests using a series of temperature and humidity combinations. After being placed for different days under light and various temperature and humidity conditions, the samples were taken out. The related substances (impurities) were detected by high performance liquid chromatography. The resulting solid was compared with the initial free base crystal form E, showing that there was no significant increase or only a slight increase in impurities. After being placed for different days under light conditions, the samples were taken out, and impurities increased. The results are shown in Table 10.

The samples taken out on the last day under light and various temperature and humidity conditions were subjected to XRPD detection, showing that the free base crystal form E had no change and had good stability.

**Table 10. Results of influence factors after drying treatment of free base crystal form E**

| Influence factor | Content (%) of the compound of formula I |
|---|---|
| 0 day | 97.45 |
| 0.5 ICH | 96.07 |
| 1 ICH | 94.52 |
| 25°CRH 92%-1 week | 97.29 |
| 25°CRH 92%-2 weeks | 97.5 |
| 40°CRH 75%-1 week | 97.36 |
| 40°CRH 75%-2 weeks | 97.51 |
| 60°C-1 week | 97.25 |
| 60°C-2 weeks | 97.55 |

## Claims

1. Crystal form A of a compound of formula I, wherein the X-ray powder diffraction pattern thereof represented by diffraction angle 2θ angle has characteristic peaks at 8.910, 10.273, 15.650, 18.617, 17.869 and 19.526, preferably has characteristic peaks at 8.910, 10.273, 13.730, 15.650, 17.869, 18.617, 19.526 and 23.452, more preferably has characteristic peaks at 8.910, 10.273, 13.730, 15.650, 17.869, 18.617, 19.526, 20.785, 22.193, 23.452, 25.049 and 26.590, wherein the error range of the 2θ angle is ±0.20,

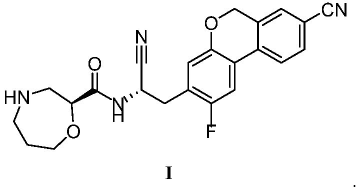

2. The crystal form A according to claim 1, wherein the differential scanning calorimetry (DSC) spectrum thereof has endothermic peaks at 116°C and 208°C, with the error range of ±2°C.

3. A method for preparing the crystal form A according to claim 1 or 2, comprising the steps of:
a) mixing the compound of formula I with Solvent A;
b) performing stirring;
wherein the Solvent A is selected from the group consisting of water, C₁₋₄ alcohol solvent, and mixed solvent of water and C₁₋₄ alcohol solvent, and the C₁₋₄ alcohol solvent preferably is methanol and ethanol.

4. Crystal form B of a compound of formula I, wherein the X-ray powder diffraction pattern thereof represented by diffraction angle 2θ angle has characteristic peaks at 6.770, 11.201, 12.989, 14.931 and 20.817, preferably has characteristic peaks at 6.770, 11.201, 12.989, 14.931, 20.817 and 25.710, more preferably has characteristic peaks at 6.770, 11.201, 12.989, 14.931, 20.817, 25.710, 31.066, 32.046 and 32.913, most preferably has characteristic peaks at 6.770, 11.201, 12.989, 14.931, 15.851, 17.228, 20.817, 22.313, 22.773, 25.710, 28.192, 31.066, 32.046 and 32.913, wherein the error range of the 2θ angle is ±0.20,

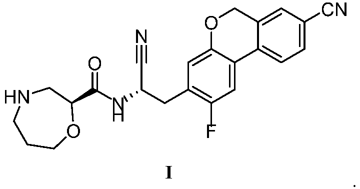

5. The crystal form B according to claim 4, wherein the DSC spectrum thereof has endothermic peaks at 114°C and 210°C, with the error range of ±2°C.

6. A method for preparing the crystal form B according to claim 4 or 5, comprising the steps of:
a) mixing the compound of formula I with Solvent B;
b) performing stirring;
wherein the Solvent B is selected from the group consisting of nitromethane and acetonitrile.

7. Crystal form C of a compound of formula I, wherein the X-ray powder diffraction pattern thereof represented by diffraction angle 2θ angle has characteristic peaks at 7.385, 10.171, 12.687, 15.902 and 19.645, preferably has characteristic peaks at 6.012, 7.385, 8.275, 10.171, 12.687, 15.120, 15.902 and 19.645, more preferably has characteristic peaks at 6.012, 7.385, 8.275, 10.171, 12.687, 15.120, 15.902, 19.645, 25.539 and 26.382, most preferably has characteristic peaks at 6.012, 7.385, 8.275, 10.171, 12.687, 13.720, 15.120, 15.902, 16.842, 19.645, 20.739, 25.539, 26.382, 27.272 and 30.887, wherein the error range of the 2θ angle is ±0.20,

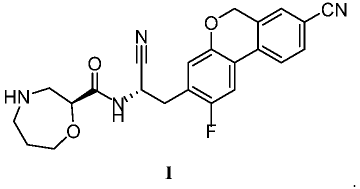

8. The crystal form C according to claim 7, wherein the DSC spectrum thereof has endothermic peaks at 76°C and 209°C, with the error range of ±2°C.

9. A method for preparing the crystal form C according to claim 7 or 8, comprising the steps of:
a) mixing the compound of formula I with Solvent C;
b) performing stirring;
wherein the Solvent C is selected from the group consisting of N,N-dimethylformamide, acetone, butanone, tetrahydrofuran and 1,2-dimethoxyethane.

10. Crystal form D of a compound of formula I, wherein the X-ray powder diffraction pattern thereof represented by diffraction angle 2θ angle has characteristic peaks at 8.477, 10.451, 13.569, 15.146, 16.335 and 17.275, preferably has characteristic peaks at 8.477, 10.451, 13.569, 15.146, 16.335, 17.275 and 18.775, more preferably has characteristic peaks at 8.477, 10.451, 13.569, 15.146, 16.335, 17.275, 18.775, 20.298, 23.990, 26.006 and 28.135, preferably has characteristic peaks at 8.477, 10.451, 13.569, 15.146, 16.335, 17.275, 18.775, 20.298, 23.093, 23.990, 24.921, 26.006, 27.047, 28.135 and 33.461, wherein the error range of the 2θ angle is ±0.20,

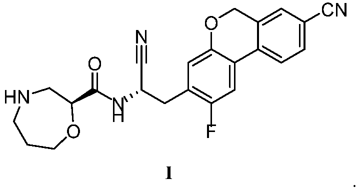

11. The crystal form D according to claim 10, wherein the DSC spectrum thereof has an endothermic peak at 211°C, with the error range of ±2°C.

12. A method for preparing the crystal form D according to claim 10 or 11, comprising the steps of:
a) mixing the compound of formula I with Solvent D;
b) performing stirring;
wherein the Solvent D is selected from the group consisting of ethyl acetate, 1,2-xylene, toluene, 1,4-dioxane and hexane.

13. Crystal form E of a compound of formula I, wherein the X-ray powder diffraction pattern thereof represented by diffraction angle 2θ angle has characteristic peaks at 8.685, 15.433, 16.654, 17.526 and 18.779, preferably has characteristic peaks at 8.685, 10.681, 13.773, 14.600, 15.433, 16.654, 17.526 and 18.779, more preferably has characteristic peaks at 8.685, 10.681, 13.773, 14.600, 15.433, 16.654, 17.526, 18.779, 19.393, 20.610, 21.653, 23.319 and 24.151, most preferably has characteristic peaks at 7.233, 8.685, 10.681, 13.773, 14.600, 15.433, 16.654, 17.526, 18.779, 20.610, 21.653, 23.319, 24.151, 25.111 and 26.192, wherein the error range of the 2θ angle is ±0.20,

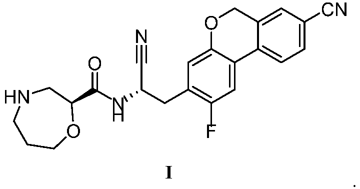

14. The crystal form E according to claim 13, wherein the DSC spectrum thereof has an endothermic peak at 207°C, with the error range of ±2°C.

15. A method for preparing the crystal form E according to claim 13 or 14, comprising a step of drying or heating the compound of formula I, wherein the temperature for drying or heating is preferably from 100°C to 200°C.

16. A pharmaceutical composition comprising the crystal form according to any one of claims 1-2, 4-5, 7-8, 10-11 and 13-14, and a pharmaceutically acceptable excipient.

17. Use of the crystal form according to any one of claims 1-2, 4-5, 7-8, 10-11 and 13-14 or the pharmaceutical composition according to claim 16, in the preparation of a medicament for preventing and/or treating asthma, obstructive pulmonary disease, bronchiectasis, ANCA-associated vasculitis, psoriasis, α1-antitrypsin deficiency, lupus nephritis, diabetes, inflammatory bowel disease, rheumatoid arthritis, nasosinusitis, hidradenitis suppurativa, or cancer.
